# EUROPÄISCHE PATENTSCHRIFT

(11) **EP 1 856 055 B1**
(45) Veröffentlichungstag und Bekanntmachung des Hinweises auf die Patenterteilung: **01.10.2014**
(21) Anmeldenummer: 06708289.1
(22) Anmeldetag: 15.02.2006
(51) Int. Cl.: C07D 231/14, A01N 43/56

(54) **PYRAZOLCARBONSÄUREANILIDE, VERFAHREN ZU IHRER HERSTELLUNG UND SIE ENTHALTENDE MITTEL ZUR BEKÄMPFUNG VON SCHADPILZEN**
PYRAZOLE CARBOXYLIC ACID ANILIDES, METHOD FOR THE PRODUCTION THEREOF AND AGENTS CONTAINING THEM FOR CONTROLLING PATHOGENIC FUNGI
ANILIDES D'ACIDE CARBOXYLIQUE PYRAZOLE, PROCÉDÉS DE PRODUCTION ASSOCIÉS ET AGENTS LES CONTENANT POUR LA LUTTE ANTIFONGIQUE

(30) Priorität: 16.02.2005 DE 102005007160
(43) Veröffentlichungstag der Anmeldung: 21.11.2007
(73) Patentinhaber: BASF SE, 67056 Ludwigshafen (DE)
(72) Erfinder: GEWEHR, Markus, 56288 Kastellaun (DE); DIETZ, Jochen, 68167 Mannheim (DE); GROTE, Thomas, 67157 Wachenheim (DE); BLETTNER, Carsten, Hong Kong (CN); GRAMMENOS, Wassilios, 67071 Ludwigshafen (DE); HÜNGER, Udo, 68167 Mannheim (DE); MÜLLER, Bernd, 67227 Frankenthal (DE); SCHIEWECK, Frank, 67258 Hessheim (DE); SCHWÖGLER, Anja, 68165 Mannheim (DE); LOHMANN, Jan Klaas, 68167 Mannheim (DE); RHEINHEIMER, Joachim, 67063 Ludwigshafen (DE); SCHÄFER, Peter, 67308 Ottersheim (DE); STRATHMANN, Siegfried, 67117 Limburgerhof (DE); STIERL, Reinhard, 67251 Freinsheim (DE)
(86) Internationale Anmeldenummer: PCT/EP2006/050962
(87) Internationale Veröffentlichungsnummer: WO 2006/087343

(56) Entgegenhaltungen:
- WO-A-00/14071
- WO-A-2004/103975
- WO-A-2005/123689
- WO-A-2005/123690
- PATENT ABSTRACTS OF JAPAN Bd. 2002, Nr. 02, 2. April 2002 (2002-04-02) & JP 2001 302605 A (SUMITOMO CHEM CO LTD), 31. Oktober 2001 (2001-10-31) in der Anmeldung erwähnt

## Beschreibung

Die vorliegende Erfindung betrifft Pyrazolcarbonsäureanilide der Formel 1 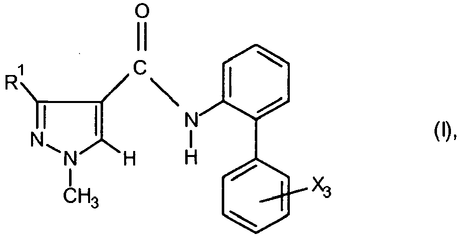 in der
- X: Fluor oder Chlor, wobei die Reste X verschiedene Bedeutungen haben können, und
- R¹: Difluormethyl oder Trifluormethyl
bedeuten.

Außerdem betrifft die Erfindung Verfahren zur Herstellung dieser Verbindungen, sie enthaltende Mittel und Verfahren zu deren Verwendung zur Bekämpfung von Schadpilzen, insbesondere Botrytis.

Aus der Literatur sind Pyrazolcarbonsäureanilide mit fungizider Wirkung bekannt. So werden beispielsweise in der EP-A 545 099 und EP-A 589 301 Biphenylanilide diesen Typs beschrieben, die an der Biphenyl-Gruppe eine Monosubstitution aufweisen.

In der WO 00/14071 werden spezifische 1,3-Dimethyl-5-fluorpyrazolcarbonsäureanilide beschrieben und deren fungizide Wirkung.

Aus der WO 03/070705 und der JP-A 2001/302605 sind Pyrazolcarbonsäureanilide bekannt, die in der Biphenyl-Gruppe eine spezifische 3fach Substitution aufweisen.

Gegenstand der WO 2004/103975 sind u.a. lodpyrazolcarboxanilide, die sich von den vorliegenden Verbindungen I insbesondere durch einen Iod-Substituenten anstelle von R¹ unterscheiden.

Aufgabe der vorliegenden Erfindung war es, Pyrazolcarbonsäureanilide mit verbesserter fungizider Wirkung aufzufinden als die Verbindungen des Standes der Technik. Demgemäß wurden die eingangs definierten Verbindungen I gefunden. Außerdem wurden Verfahren zur Herstellung dieser Verbindungen, sie enthaltende Mittel und Verfahren zu deren Verwendung zur Bekämpfung von Schadpilzen gefunden.

Die Verbindungen der Formel I weisen eine gegenüber den bekannten Verbindungen verbesserte Wirksamkeit gegen Schadpilze auf.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Man erhält die Verbindungen I im allgemeinen dadurch, dass man ein Carbonsäurehalogenid der Formel II in an sich bekannter Weise (z. B. J. March, Advanced Organic Chemistry, 2nd Ed., 382 f., McGraw-Hill, 1977) in Gegenwart einer Base mit einem Anilin der Formel 111 umsetzt:

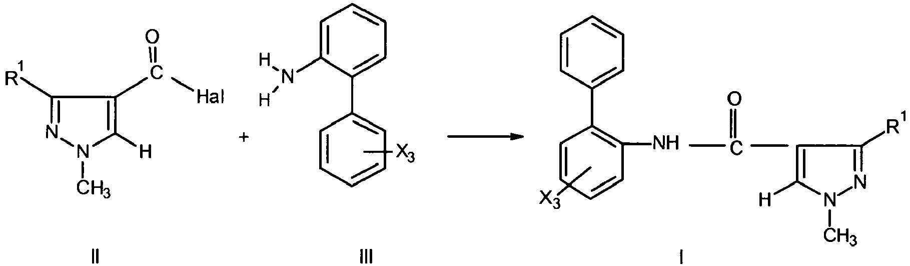

Der Rest Hal in der Formel I I steht für ein Halogenatom wie Fluor, Chlor, Brom und Jod, insbesondere Fluor, Chlor oder Brom. Diese Umsetzung erfolgt üblicherweise bei Temperaturen von (-20)°C bis 100°C, vorzugsweise 0°C bis 50°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Methylenchlorid, Dimethylsulfoxid und Dimethylformamid, besonders bevorzugt Toluol, Methylenchlorid und Tetrahydrofuran.

Es können auch Gemische der genannten Lösungsmittel verwendet werden.

Als Basen kommen allgemein anorganische Verbindungen, z.B. Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat und metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z. B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Triethylamin und Pyridin verwendet.

Die Basen werden im allgemeinen in äquimolaren Mengen bezogen auf die Verbindung II eingesetzt. Sie können aber auch in einem Überschuß von 5 mol-% bis 30 mol-%, vorzugsweise 5 mol-% bis 10 mol-%, oder - im Falle der Verwendung von tertiären Aminen - gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß von 1 mol-% bis 20 mol-%, vorzugsweise 1 mol-% bis 10 mol-%, bezogen auf III einzusetzen.

Die für die Herstellung der Verbindungen I benötigten Ausgangsstoffe der Formel II und III sind bekannt oder können analog zu den bekannten Verbindungen synthetisiert werden (Helv. Chim. Acta, 60, 978 (1977); Zh. Org. Khim., 26, 1527 (1990); Heterocycles 26, 1885 (1987); Izv. Akad. Nauk. SSSR Ser. Khim., 2160 (1982); THL 28, 593 (1987); THL 29, 5463 (1988)).

Weiterhin wurde gefunden, dass man Verbindungen der Formel I dadurch erhält, dass man in bekannter Weise Carbonsäuren der Formel IV mit einem Anilin der Formel III in Gegenwart von Dehydratisierungsmitteln und ggf. einer organischen Base umsetzt.

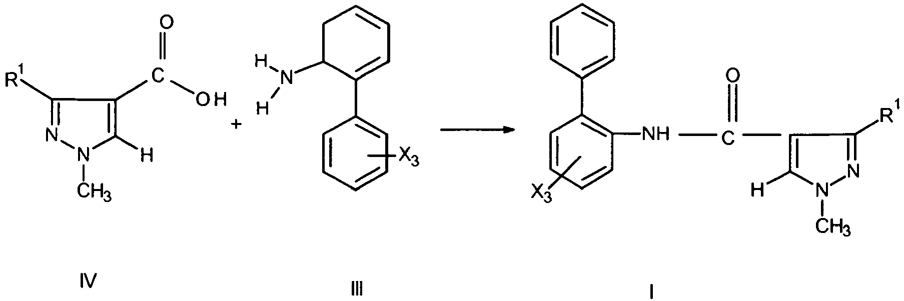

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Methylenchlorid, Toluol und Tetrahydrofuran.

Es können auch Gemische der genannten Lösungsmittel verwendet werden.

Als Dehydratisierungsmittel kommen 1,1'-Carbonyldiimidazol, Bis(2-oxo-3-oxazolidinyl)phosphorylchlorid, Carbodiimide wie N,N'-Dicyclohexylcarbodiimid und N-(3-Dimethylaminopropyl)-N'-ethylcarbodiimid, Phosphoniumsalze wie (Benzotriazol-1-yloxy)tris(dimethylamino)phosphoniumhexafluorphosphat, Bromtripyrrolidinophosphoniumhexafluorphosphat, Bromtris(dimethylamino)phosphoniumhexafluorphosphat und Chlortripyrrolidinophosphoniumhexafluorphosphat, Uronium- und Thiuroniumsalze wie O-(Benzotriazol-1-yl)-N,N,N',N'-tetramethyluroniumhexafluorphosphat, O-(7-Azabenzotriazol-1-yl)-N,N,N',N'-tetramethyluroniumhexafluorphosphat, S-(1-Oxido-2-pyridyl)-N,N,N',N'-tetramethylthiuroniumtetrafluorborat, O-(2-Oxo-1(2H)pyridyl)-N,N,N',N'-tetramethyluroniumtetrafluorborat, O-[(Ethoxycarbonyl)cyano-methylenamino]-N,N,N',N'-tetramethyluroniumtetrafluorborat, Carbeniumsalze wie (Benzotriazol-1-yloxy)dipyrrolidinocarbeniumhexafluorphosphat, (Benzotriazol-1-yloxy)dipiperidinocarbeniumhexafluorphosphat, O-(3,4-Dihydro-4-oxo-1,2,3-benzotriazin-3-yl)-N,N,N',N'-tetramethyluroniumtetrafluorborat, Chlor-N',N'-bis(tetramethylen)formamidiniumtetrafluorborat, Chlordipyrrolidinocarbeniumhexafluorphosphat, Chlor-N,N,N',N'-bis(pentamethylen)formamidiniumtetrafluorborat, Imidazoliumsalze wie 2-Chlor-1,3-dimethylimidazolidiniumtetrafluorborat, vorzugsweise 1,1 '-Carbonyldiimidazol, Bis(2-oxo-3-oxazolidinyl)phosphorylchlorid, N,N'-Dicyclohexylcarbodiimid und N-(3-Dimethylaminopropyl)-N'-ethylcarbodiimid, in Betracht.

Als organische Basen kommen tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin verwendet. Die Basen werden im allgemeinen im Überschuss von 10 mol% bis 200 mol%, vorzugsweise von 50 mol% bis 150 mol-% bezogen auf die Verbindung IV eingesetzt.

Die Edukte werden im allgemeinen in äquimolaren Mengen miteinander umgesetzt. Es kann für die Ausbeute vorteilhaft sein, eine der Verbindungen in einem Überschuß von 1 mol-% bis 20 mol-%, vorzugsweise 1 mol% bis 10 mol-%, einzusetzen. Die Dehydratisierungsmittel werden im allgemeinen im Überschuß von 5 mol-% bis 100 mol-%, vorzugsweise 5 mol% bis 60 mol%, eingesetzt.

Die für die Herstellung der Verbindungen I benötigten Ausgangsstoffe der Formel III und IV sind bekannt oder können analog zu den bekannten Verbindungen synthetisiert werden.

Im Hinblick auf die biologische Wirkung der Verbindungen I sind die folgenden Bedeutungen der Variablen bevorzugt, und zwar jeweils für sich allein oder in Kombination:
- X: Fluor;
- R¹: Difluormethyl.

Die Reste X stehen vorzugsweise in 2,4,5- oder 3,4,5-Position, insbesondere in 3,4,5-Position.

Insbesondere sind im Hinblick auf ihre Verwendung als Fungizide die Verbindungen der allgemeinen Formeln I-A bevorzugt: 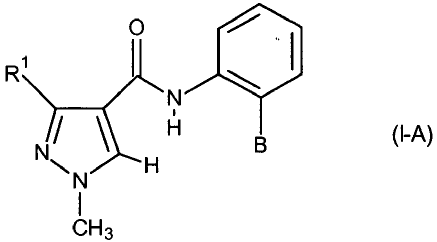 Tabelle A

**Tabelle 1:**

| Verbindungen der allgemeinen Formel I-A, worin R¹ und B für jede einzelne Verbindung jeweils einer Zeile der Tabelle A entsprechen. | | |
|---|---|---|
| **Nr.** | **B** | **R¹** |
| 23 | 2,3,4-Trichlorphenyl | CF₃ |
| 24 | 2,3,5-Trichlorphenyl | CF₃ |
| 25 | 2,3,6-Trichlorphenyl | CF₃ |

| **Nr.** | **B** | **R¹** |
|---|---|---|
| 26 | 2,4,5-Trichlorphenyl | CF₃ |
| 27 | 2,4,6-Trichlorphenyl | CF₃ |
| 28 | 3,4,5-Trichlorphenyl | CF₃ |
| 29 | 2,3,4-Trifluorphenyl | CF₃ |
| 30 | 2,3,5-Trifluorphenyl | CF₃ |
| 31 | 2,3,6-Trifluorphenyl | CF₃ |
| 32 | 2,4,5-Trifluorphenyl | CF₃ |
| 33 | 2,4,6-Trifluorphenyl | CF₃ |
| 34 | 3,4,5-Trifluorphenyl | CF₃ |
| 35 | 2-Chlor-3,4-difluorphenyl | CF₃ |
| 36 | 2-Chlor-4,5-difluorphenyl | CF₃ |
| 37 | 2-Chlor-5,6-difluorphenyl | CF₃ |
| 38 | 2-Chlor-3,5-difluorphenyl | CF₃ |
| 39 | 2-Chlor-3,6-difluorphenyl | CF₃ |
| 40 | 2-Chlor-4,6-difluorphenyl | CF₃ |
| 41 | 3-Chlor-2,4-difluorphenyl | CF₃ |
| 42 | 3-Chlor-2,5-difluorphenyl | CF₃ |
| 43 | 3-Chlor-2,6-difluorphenyl | CF₃ |
| 44 | 3-Chlor-4,5-difluorphenyl | CF₃ |
| 45 | 3-Chlor-4,6-difluorphenyl | CF₃ |
| 46 | 3-Chlor-5,6-difluorphenyl | CF₃ |
| 47 | 4-Chlor-2,3-difluorphenyl | CF₃ |
| 48 | 4-Chlor-2,5-difluorphenyl | CF₃ |
| 49 | 4-Chlor-2,6-difluorphenyl | CF₃ |
| 50 | 4-Chlor-3,5-difluorphenyl | CF₃ |
| 51 | 2-Fluor-3,4-dichlorphenyl | CF₃ |
| 52 | 2-Fluor-4,5-dichlorphenyl | CF₃ |
| 53 | 2-Fluor-5,6-dichlorphenyl | CF₃ |
| 54 | 2-Fluor-3,5-dichlorphenyl | CF₃ |
| 55 | 2-Fluor-3,6-dichlorphenyl | CF₃ |
| 56 | 2-Fluor-4,6-dichlorphenyl | CF₃ |
| 57 | 3-Fluor-2,4-dichlorphenyl | CF₃ |
| 58 | 3-Fluor-2,5-dichlorphenyl | CF₃ |
| 59 | 3-Fluor-2,6-dichlorphenyl | CF₃ |
| 60 | 3-Fluor-4,5-dichlorphenyl | CF₃ |
| 61 | 3-Fluor-4,6-dichlorphenyl | CF₃ |
| 62 | 3-Fluor-5,6-dichlorphenyl | CF₃ |
| 63 | 4-Fluor-2,3-dichlorphenyl | CF₃ |
| 64 | 4-Fluor-2,5-dichlorphenyl | CF₃ |
| 65 | 4-Fluor-2,6-dichlorphenyl | CF₃ |
| 66 | 4-Fluor-3,5-dichlorphenyl | CF₃ |
| 89 | 2,3,4-Trichlorphenyl | CHF₂ |
| 90 | 2,3,5-Trichlorphenyl | CHF₂ |
| 91 | 2,3,6-Trichlorphenyl | CHF₂ |
| 92 | 2,4,5-Trichlorphenyl | CHF₂ |
| 93 | 2,4,6-Trichlorphenyl | CHF₂ |
| 94 | 3,4,5-Trichlorphenyl | CHF₂ |
| 95 | 2,3,4-Trifluorphenyl | CHF₂ |
| 96 | 2,3,5-Trifluorphenyl | CHF₂ |
| 97 | 2,3,6-Trifluorphenyl | CHF₂ |
| 98 | 2,4,5-Trifluorphenyl | CHF₂ |
| 99 | 2,4,6-Trifluorphenyl | CHF₂ |
| 100 | 3,4,5-Trifluorphenyl | CHF₂ |
| 101 | 2-Chlor-3,4-difluorphenyl | CHF₂ |
| 102 | 2-Chlor-4,5-difluorphenyl | CHF₂ |
| 103 | 2-Chlor-5,6-difluorphenyl | CHF₂ |
| 104 | 2-Chlor-3,5-difluorphenyl | CHF₂ |
| 105 | 2-Chlor-3,6-difluorphenyl | CHF₂ |
| 106 | 2-Chlor-4,6-difluorphenyl | CHF₂ |
| 107 | 3-Chlor-2,4-difluorphenyl | CHF₂ |
| 108 | 3-Chlor-2,5-difluorphenyl | CHF₂ |
| 109 | 3-Chlor-2,6-difluorphenyl | CHF₂ |
| 110 | 3-Chlor-4,5-difluorphenyl | CHF₂ |
| 111 | 3-Chlor-4,6-difluorphenyl | CHF₂ |
| 112 | 3-Chlor-5,6-difluorphenyl | CHF₂ |
| 113 | 4-Chlor-2,3-difluorphenyl | CHF₂ |
| 114 | 4-Chlor-2,5-difluorphenyl | CHF₂ |
| 115 | 4-Chlor-2,6-difluorphenyl | CHF₂ |
| 116 | 4-Chlor-3,5-difluorphenyl | CHF₂ |
| 117 | 2-Fluor-3,4-dichlorphenyl | CHF₂ |
| 118 | 2-Fluor-4,5-dichlorphenyl | CHF₂ |
| 119 | 2-Fluor-5,6-dichlorphenyl | CHF₂ |
| 120 | 2-Fluor-3,5-dichlorphenyl | CHF₂ |
| 121 | 2-Fluor-3,6-dichlorphenyl | CHF₂ |
| 122 | 2-Fluor-4,6-dichlorphenyl | CHF₂ |
| 123 | 3-Fluor-2,4-dichlorphenyl | CHF₂ |
| 124 | 3-Fluor-2,5-dichlorphenyl | CHF₂ |
| 125 | 3-Fluor-2,6-dichlorphenyl | CHF₂ |
| 126 | 3-Fluor-4,5-dichlorphenyl | CHF₂ |
| 127 | 3-Fluor-4,6-dichlorphenyl | CHF₂ |
| 128 | 3-Fluor-5,6-dichlorphenyl | CHF₂ |
| 129 | 4-Fluor-2,3-dichlorphenyl | CHF₂ |
| 130 | 4-Fluor-2,5-dichlorphenyl | CHF₂ |
| 131 | 4-Fluor-2,6-dichlorphenyl | CHF₂ |
| 132 | 4-Fluor-3,5-dichlorphenyl | CHF₂ |

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Peronosporomyceten (syn. Oomyceten)* und *Basidiomyceten.* Sie sind zum Teil systemisch wirksam und können im Pflanzenschutz als Blatt-, Boden- und Beizfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- *Alternaria* Arten an Gemüse, Raps, Zuckerrüben und Obst und Reis (z.B. *A. solani* oder *A. alternata* an Kartoffel und anderen Pflanzen),
- *Aphanomyces* Arten an Zuckerrüben und Gemüse,
- *Bipolaris-* und *Drechstera* Arten an Mais, Getreide, Reis und Rasen (z.B. *D. teres* an Gerste, *D. tritci-repentis* an Weizen),
- *Blumeria graminis* (Echter Mehltau) an Getreide,
- *Botrytis cinerea* (Grauschimmel) an Erdbeeren, Gemüse, Blumen und Weinreben,
- *Bremia lactucae* an Salat,
- *Cercospora* Arten an Mais, Sojabohnen, Reis und Zuckerrüben (z.B. *C. beticula* an Zuckerrüben),
- *Cochliobolus* Arten an Mais, Getreide, Reis (z.B. *Cochliobolus sativus* an Getreide, *Cochliobolus miyabeanus* an Reis),
- *Colletotricum* Arten an Sojabohnen, Baumwolle und anderen Pflanzen (z.B. *C. acutatum* an verschiedenen Pflanzen),
- *Exserohilum* Arten an Mais,
- *Erysiphe cichoracearumund Sphaerotheca fuliginea* an Gurkengewächsen,
- *Fusarium* und *Verticillium* Arten (z.B. *V. dahliae*) an verschiedenen Pflanzen (z.B. *F. graminearum* an Weizen),
- *Gaeumanomyces graminis* an Getreide,
- *Gibberella* Arten an Getreide und Reis (z.B. *Gibberella fujikuroi* an Reis),
- *Grainstaining complex* an Reis,
- *Helminthosporium* Arten (z.B. *H. graminicola*) an Mais und Reis,
- *Michrodochium nivale* an Getreide,
- *Mycosphaerella* Arten an Getreide, Bananen und Erdnüssen *(M. graminicola* an Weizen, *M. fijiesis* an Banane),
- *Phakopsara pachyrhizi* und *Phakopsara meibomiae* an Sojabohnen,
- *Phomopsis* Arten an Sojabohnen, Sonnenblumen und Weinreben (*P. viticola* an Weinreben, *P. helianthü* an Sonnenblumen),
- *Phytophthora infestans* an Kartoffeln und Tomaten,
- *Plasmopara viticola* an Weinreben,
- *Podosphaera leucotricha* an Apfel,
- *Pseudocercosporella herpotrichoides* an Getreide,
- *Pseudoperonospora* Arten an Hopfen und Gurkengewächsen (z.B. *P. cubenis* an Gurke),
- *Puccinia* Arten an Getreide, Mais und Spargel (*P. triticina und P. striformis an Weizen, P. asparagi* an Spargel),
- *Pyrenophora* Arten an Getreide,
- *Pyricularia oryzae, Corticium sasakii, Sarociadium oryzae, S.attenuatum, Entyloma oryzae* an Reis,
- *Pyricularia grisea* an Rasen und Getreide,
- *Pythium spp.* an Rasen, Reis, Mais, Baumwolle, Raps, Sonnenblumen, Zuckerrüben, Gemüse und anderen Pflanzen,
- *Rhizoctonia-Arten* (z.B. *R. solani*) an Baumwolle, Reis, Kartoffeln, Rasen, Mais, Raps, Kartoffeln, Zuckerrüben, Gemüse und anderen Pflanzen,
- *Sclerotinia* Arten (z.B. *S. sclerotiorum*) an Raps, Sonnenblumen und anderen Pflanzen,
- *Septoria tritici* und *Stagonospora nodorum* an Weizen,
- *Erysiphe* (syn. *Uncinulanecator*) an Weinrebe,
- *Setospaeria* Arten an Mais und Rasen,
- *Sphacelotheca reilinia* an Mais,
- *Thievaliopsis* Arten an Sojabohnen und Baumwolle,
- *Tilletia Arten* an Getreide,
- *Ustilago* Arten an Getreide, Mais und Zuckerrübe und
- *Venturia* Arten (Schorf) an Apfel und Birne (z.B. *V. inaequalis* an Apfel).

Die Verbindungen I eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomyceten wie *Coniophora* spp., *Coriolus* spp., *Gloeophyllum* spp., *Lentinus* spp., *Pleurotus* spp., *Poria* spp., *Serpula* spp. und *Tyromyces* spp., Deuteromyceten wie *Aspergillus* spp., *Cladosporium* spp., *Penicillium* spp., *Trichoderma* spp., *Alternaria* spp., *Paecilomyces* spp. und Zygomyceten wie *Mucorspp.,* darüber hinaus im Materialschutz folgende Hefepilze: *Candida* spp. und *Saccharomyces cerevisae.*

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung, z.B. durch Bestäuben, Beschichten oder Tränken von Saatgut, werden im allgemeinen Wirkstoffmengen von 1 bis 1000 g/100 kg, vorzugsweise 5 bis 100 g/100 kg, Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im Wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkyl-arylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpoly-glykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Formulierungen für die Saatgutbehandlung können zusätzlich Bindemittel und/oder Geliermittel und gegebenenfalls Farbstoffe enthalten.

Bindemittel können zugesetzt werden, um Haftung der Wirkstoffe auf dem Saatgut nach der Behandlung zu erhöhen. Geeignete Bindemittel sind beispielsweise EO/PO Blockcopolymer-Tenside, aber auch Polyvinylalcohole, Ppolyvinylpyrrolidone, Polyacrylate, Polymethacrylate, Polybutene, Polyisobutylene, Polystyrole, Polyethylenamine, Polyethylenamide, Polyethylenimine (Lupasol^{®}, Polymin^{®}), Polyether, Polyurethane, Polyvinylacetate, Tylose und Copolymere aus diesen Polymeren. Ein geeignetes Geliermittel ist beispielsweise Carrageen (Satiagel^{®}).

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Die Wirkstoffkonzentrationen in den anwendungsfertigen Zubereitungen können in größeren Bereichen variiert werden. Im Allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Für die Saatgutbehandlung ergeben die betreffenden Formulierungen nach zwei- bis zehnfacher Verdünnung Wirkstoffkonzentrationen von 0,01 bis 60 Gew.-%, bevorzugt 0,1 bis 40 Gew.-% in den fertig verwendbaren Zubereitungen.

### Beispiele für Formulierungen sind: 1. Produkte zur Verdünnung in Wasser

### A) Wasserlösliche Konzentrate (SL)

10 Gew.-Teile einer erfindungsgemäßen Verbindung I werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit einem Wirkstoffgehalt von 10 Gew.-%.

### B) Dispergierbare Konzentrate (DC)

20 Gew.-Teile einer erfindungsgemäßen Verbindung I werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%.

### C) Emulgierbare Konzentrate (EC)

15 Gew.-Teile einer erfindungsgemäßen Verbindung I werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat einen Wirkstoffgehalt von 15 Gew.-%.

### D) Emulsionen (EW, EO)

25 Gew.-Teile einer erfindungsgemäßen Verbindung I werden in 35 Gew.-Teilen Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E) Suspensionen (SC, OD)

20 Gew.-Teile einer erfindungsgemäßen Verbindung I werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

### F) Wasserdispergierbare und wasserlösliche Granulate (WG, SG)

50 Gew.-Teile einer erfindungsgemäßen Verbindung I werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G) Wasserdispergierbare und wasserlösliche Pulver (WP, SP)

75 Gew.-Teile einer erfindungsgemäßen Verbindung I werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### 2. Produkte für die Direktapplikation

### H) Stäube (DP)

5 Gew.-Teile einer erfindungsgemäßen Verbindung I werden fein gemahlen und mit 95 Gew.-Teilen feinteiligem Kaolin innig vermischt. Man erhält dadurch ein Stäubemittel mit einem Wirkstoffgehalt von 5 Gew.-%.

### J) Granulate (GR, FG, GG, MG)

0,5 Gew-Teile einer erfindungsgemäßen Verbindung I werden fein gemahlen und mit 99,5 Gew.-Teilen Trägerstoffe verbunden. Gängige Verfahren sind dabei die Extrusion, die Sprühtrocknung oder die Wirbelschicht. Man erhält dadurch ein Granulat für die Direktapplikation mit einem Wirkstoffgehalt von 0,5 Gew.-%.

### K) ULV- Lösungen (UL)

10 Gew.-Teile einer erfindungsgemäßen Verbindung I werden in 90 Gew.-Teilen eines organischen Lösungsmittels, z.B. Xylol, gelöst. Dadurch erhält man ein Produkt für die Direktapplikation mit einem Wirkstoffgehalt von 10 Gew.-%.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubmitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können üblicherweise zu den erfindungsgemäßen Mitteln im Gewichtsverhältnis 1:100 bis 100:1, bevorzugt 1:10 bis 10:1 gemischt werden.

Als Adjuvants in diesem Sinne kommen insbesondere in Frage: organisch modifizierte Polysiloxane, z.B. Break Thru S 240^{®}; Alkoholalkoxylate, z. B. Atplus 245^{®}, Atplus MBA 1303^{®}, Plurafac LF 300^{®} und Lutensol ON 30^{®}; EO-PO-Blockpolymerisate, z. B. Pluronic RPE 2035^{®} und Genapol B^{®}; Alkoholethoxylate, z. B. Lutensol XP 80^{®}; und Natriumdioctylsulfosuccinat, z. B. Leophen RA^{®}.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren wie Prohexadion-Ca, Fungiziden oder auch mit Düngemitteln. Beim Vermischen der Verbindungen I bzw. der sie enthaltenden Mittel mit einem oder mehreren weiteren Wirkstoffen, insbesondere Fungiziden, kann in vielen Fällen das Wirkungsspektrum verbreitet oder Resistenzentwicklungen vorgebeugt werden. In vielen Fällen erhält man dabei synergistische Effekte.

Die folgende Liste von Fungiziden, mit denen die erfindungsgemäßen Verbindungen gemeinsam angewendet werden können, soll die Kombinationsmöglichkeiten erläutern, nicht aber einschränken:
Strobilurine
   - Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl)-benzyl)-carbaminsäuremethyl ester, 2-(ortho-(2,5-Dimethylphenyl-oxymethylen)phenyl)-3-methoxyacrylsäuremethylester;
Carbonsäureamide
   - Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure-(2-cyano-phenyl)-amid;
   - Carbonsäuremorpholide: Dimethomorph, Flumorph;
   - Benzoesäureamide: Flumetover, Fluopicolide (Picobenzamid), Zoxamide;
   - Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;
Azole
   - Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazol, Myclobutanil, Penconazole, Propiconazole, Prothioconazole, Simeconazole, Tebuconazole, Tetraconazole, Triadimenol, Triadimefon, Triticonazole;
   - Imidazole: Cyazofamid, Imazalil, Pefurazoate, Prochloraz, Triflumizole;
   - Benzimidazole: Benomyl, Carbendazim, Fuberidazole, Thiabendazole;
   - Sonstige: Ethaboxam, Etridiazole, Hymexazole;
Stickstoffhaltige Heterocyclylverbindungen
   - Pyridine: Fluazinam, Pyrifenox, 3-[5-(4-Chlor-phenyl)-2,3-dimethyl-isoxazolidin-3-yl]-pyridin;
   - Pyrimidine: Bupirimate, Cyprodinil, Ferimzone, Fenarimol, Mepanipyrim, Nuarimol, Pyrimethanil;
   - Piperazine: Triforine;
   - Pyrrole: Fludioxonil, Fenpiclonil;
   - Morpholine: Aldimorph, Dodemorph, Fenpropimorph, Tridemorph;
   - Dicarboximide: Iprodione, Procymidone, Vinclozolin;
   - sonstige: Acibenzolar-S-methyl, Anilazin, Captan, Captafol, Dazomet, Diclomezine Fenoxanil, Folpet, Fenpropidin, Famoxadone, Fenamidone, Octhilinone, Probenazole, Proquinazid, Pyroquilon, Quinoxyfen, Tricyclazole, 5-Chlor-7-(4-methylpiperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;
Carbamate und Dithiocarbamate
   - Dithiocarbamate: Ferbam, Mancozeb, Maneb, Metiram, Metam, Propineb, Thiram, Zineb, Ziram;
   - Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester;
Sonstige Fungizide
   - Guanidine: Dodine, Iminoctadine, Guazatine;
   - Antibiotika: Kasugamycin, Polyoxine, Streptomycin, Validamycin A;
   - Organometallverbindungen: Fentin Salze;
   - Schwefelhaltige Heterocyclylverbindungen: Isoprothiolane, Dithianon;
   - Organophosphorverbindungen: Edifenphos, Fosetyl, Fosetyl-aluminium, Iprobenfos, Pyrazophos, Tolclofos-methyl, Phosphorige Säure und ihre Salze;
   - Organochlorverbindungen: Thiophanate Methyl, Chlorothalonil, Dichlofluanid, Tolylfluanid, Flusulfamide, Phthalide, Hexachlorbenzene, Pencycuron, Quintozene;
   - Nitrophenylderivate: Binapacryl, Dinocap, Dinobuton;
   - Anorganische Wirkstoffe: Bordeaux Brühe, Kupferacetat, Kupferhydroxid, Kupferoxychlorid, basisches Kupfersulfat, Schwefel;
   - Sonstige: Spiroxamine, Cyflufenamid, Cymoxanil, Metrafenon.

### Synthesebeispiele

### Beispiel 1 (nicht erfindungsgemäß):

### 1,3-Dimethyl-1H-pyrazol-4-carbonsäure (3'-chlor-4'-fluorbiphenyl-2-yl)-amid

Zu einer Lösung von 0,30 g 1,3-Dimethyl-1*H*-pyrazol-4-carbonsäure und 0,43 g Triethylamin in 30 ml Dichlormethan wurden bei Raumtemperatur 0,47 g 3'-Chlor-4'-fluor-2-aminobiphenyl und 0,82 g Bis-(2-oxo-3-oxazolidinyl)phosphorylchlorid gegeben. Die Mischung wurde 12 Stunden bei Raumtemperatur gerührt. Danach wurde sukzessive zweimal mit verdünnter Salzsäure, zweimal mit wässriger Natriumhydrogencarbonatlösung und einmal mit Wasser gewaschen. Die organische Phase wurde getrocknet und eingeengt. Das Rohprodukt wurde durch Säulenchromatographie mit Cyclohexan/Methyl-*tert*-butylether 1:2 an Kieselgel gereinigt. Man erhielt dadurch 0,56 g des gewünschten Produktes als weiße Kristalle vom Fp. 177-180°C.

### Beispiel 2 (nicht erfindungsgemäß):

### 3-Fluormethyl-1-methyl-1H-pyrazol-4-carbonsäure (3'-chlor-4'-fluorbiphenyl-2-yl)-amid

Zu einer Lösung von 0,33 g 3'-Chlor-4'-fluor-2-aminobiphenyl und 0,18 g Pyridin in 10 ml Toluol wurden bei Raumtemperatur 0,27 g 3-Fluormethyl-1-methyl-1*H*-pyrazol-4-carbonsäurechlorid getropft und das Gemisch 16 Stunden bei Raumtemperatur nachgerührt. Es wurden 10 ml Tetrahydrofuran und 30 ml Methyl-*tert*-butylether zugesetzt und die organische Phase wurde sukzessive mit 2%iger Salzsäure, zweimal mit 2%iger Natronlauge und anschließend mit verdünnter wässriger Natriumchloridlösung gewaschen. Die organische Phase wurde getrocknet und im Vakuum eingeengt. Das Rohprodukt wurde mit 10 ml Diisopropylether verrührt, der zurückgebliebene Feststoff abgetrennt und getrocknet. Dabei erhielt man 0,46 g des gewünschten Produkts als weißes Pulver vom Fp. 133-134°C.

### Beispiel 3 (nicht erfindungsgemäß):

### 1-Methyl-3-trifluormethyl-1H-pyrazol-4-carbonsäure (3',4'-dichlorbiphenyl-2-yl)-methylamid

Zu einer Lösung von 0,02 g Natriumhydrid in 5 ml N,N-Dimethylformamid wurden unter Eiskühlung 0,25 g 1-Methyl-3-trifluormethyl-1*H*-pyrazol-4-carbonsäure (3',4'-dichlorbiphenyl-2-yl)-amid und 0,09 g Methyljodid gegeben. Die Mischung wurde 12 Stunden bei Raumtemperatur nachgerührt und anschließend mit 1%iger Salzsäure versetzt und Methyl-tert-butylether versetzt. Die organische Phase wurde sukzessive mit Wasser und gesättigter wässriger Natriumchloridlösung gewaschen und die Lösung im Vakuum eingeengt. Das Rohprodukt wurde durch Säulenchromatographie mit Cyclohexan/Ethylacetat 1:1 an Kieselgel gereinigt. Man erhielt dadurch 0,15 g des gewünschten Produktes als milchiges Öl.

### Beispiel 4 (nicht erfindungsgemäß):

### 3-(Dichlorfluormethyl)-1-methyl-1H-pyrazol-4-carbonsäure (3',4'-dichlorbiphenyl-2-yl)-amid

### a) 3-(Dichlorfluormethyl)-1-methyl-1H-pyrazol-4-carbonsäure (3',4'-dichlorbiphenyl-2-yl)-amid

0,37 g des Öls aus 4b wurden tropfenweise zu einer Lösung von 0,36 g 3',4'-Dichlor-2-aminobiphenyl und 0,18 g Pyridin in 10 ml Toluol gegeben und die Reaktionsmischung wurde 16 Stunden bei Raumtemperatur nachgerührt. Danach wurden 10 ml Tetrahydrofuran und 30 ml Methyl-tert-butylether zugesetzt. Die organische Phase wurde sukzessive mit 2%iger Salzsäure, zweimal mit wässriger Natriumhydrogencarbonatlösung und mit verdünnter wässriger Natriumchloridlösung gewaschen. Die organische Phase wurde getrocknet und bei reduziertem Druck eingeengt. Das Rohprodukt wurde mit 10 ml Diisopropylether verrührt, der zurückgebliebene Feststoff abgetrennt und getrocknet. Dabei erhielt man 0,48 g des gewünschten Produkts als weißes Pulver vom Fp. 145-146°C.

### b) 3-Dichlorfluormethyl-1-methyl-4-pyrazolcarbonsäurechlorid

Eine Mischung von 5,3 g 3-Dichlorfluormethyl-1-methyl-4-pyrazolcarbonsäure und 27,8 g Thionylchlorid wurde für 2 Stunden am Rückfluß erhitzt. Das Reaktionsgemisch wurde anschließend einrotiert und zwei mal mit 50 ml Toluol codestilliert. Das isolierte Öl wurde ohne weitere Aufreinigung direkt weiter umgesetzt.

### c) 3-Dichlorfluormethyl-1-methyl-4-pyrazolcarbonsäure

Zu einer Mischung von 5,13 g Kaliumtrimethylsilanolat und 100 ml Tetrahydrofuran wurde bei Raumtemperatur eine Lösung von 10,20 g 3-Dichlorfluormethyl-1-methyl-4-carbonsäureethylester in 20 ml Tetrahydrofuran getropft und das Gemisch wurde für 12 Stunden bei Raumtemperatur nachgerührt. Der Niederschlag wurde abgetrennt, mit Tetrahydrofuran gewaschen und bei reduziertem Druck getrocknet. Der erhaltene Feststoff wurde in 200 ml Eiswasser gelöst und die Lösung mit 10%iger Salzsäure auf pH 2 eingestellt. Der Niederschlag wurde zweimal mit Methyl-*tert*-butylether extrahiert und die vereinigten organischen Phasen wurden mit gesättigter wässriger Natriumchloridlösung gewaschen. Nach Trocknen und Verdampfen des Lösungsmittels bei reduziertem Druck isolierte man 5.50 g der obigen Säure als weißes Pulver vom Fp. 167-169°C.

Nach den hier angegebenen Vorschriften wurden die in der nachfolgenden Tabelle 9 angegebenen Verbindungen der allgemeinen Formel I hergestellt.

**Tabelle 9**

| **Beispiel** | **R¹** | **X** | **Charakterisierung (Smp. oder ¹H-NMR)** |
|---|---|---|---|
| 9.7 | CF₃ | 3,4,5-F₃ | 120-124°C |
| 9.8 | CF₃ | 2,4,5-F₃ | 110-113°C |
| 9.10 | CF₃ | 2,3,4-F₃ | 123-125°C |
| 9.12 | CHF₂ | 3,4,5-F₃ | 113-116°C |
| 9.13 | CHF₂ | 2,4,5-F₃ | ¹H-NMR (CDCl₃): δ = 8.20 (d, 1H), 7.95 (s, 1H), 7.80 (brs, 1H), 7.45 (m, 1H), 7.25 (m, 2H), 7.15 (m, 1H), 7.00 (m, 1H), 6.62 (t, 1H), 3.90 (s, 3H) |

### Anwendungsbeispiele

Die fungizide Wirkung der erfindungsgemäßen Verbindungen I ließ sich durch folgende Versuche zeigen:
Die Wirkstoffe wurden als eine Stammlösung aufbereitet mit 25 mg Wirkstoff, welcher mit einem Gemisch aus Aceton und/oder Dimethylsulfoxid und dem Emulgator Uniperol^{®} EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der gewünschten Wirkstoffkonzentration verdünnt.

### Wirksamkeit gegen den Grauschimmel an Paprikablättern verursacht durch Botrytis cinerea bei protektiver Anwendung

Paprikasämlinge der Sorte "Neusiedler Ideal Elite" wurden, nachdem sich 2 - 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1.7 x 10⁶ Sporen/ml in einer 2 %igen wässrigen Biomalzlösung enthielt, inokuliert. Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

In diesem Test zeigten die mit 250 mg/l der Verbindungen 9.12 und 9.13, aus Tabelle 9 behandelten Pflanzen maximal 20 % Befall, während die unbehandelten Pflanzen zu 90 % befallen waren.

### Wirksamkeit gegen die Blattfleckenkrankheit an Weizen verursacht durch Leptosphaeria nodorum

Töpfe mit Weizenpflanzen der Sorte "Kanzler" wurden mit einer wässrigen Suspension in der unten angegebenen Wirkstoffkonzentration bis zur Tropfnässe besprüht. Am folgenden Tag wurden die Töpfe mit einer wässrigen Sporensuspension von *Leptosphaeria nodorum (syn.Stagonospora nodorum, Septoria nodorum)* inokuliert. Anschließend wurden die Pflanzen in einer Kammer bei 20°C und maximaler Luftfeuchte aufgestellt. Nach 8 Tagen hatte sich die Blattfleckenkrankheit auf den unbehandelten, jedoch infizierten Kontrollpflanzen so stark entwickelt, dass der Befall visuell in % ermittelt werden konnte.

In diesem Test zeigten die mit 250 mg/l der Verbindung 9.13 aus Tabelle 9 behandelten Pflanzen maximal 20 % Befall, während die unbehandelten Pflanzen zu 60 % befallen waren.

### Kurative Wirksamkeit gegen Weizenbraunrost verursacht durch Puccinia recondita

Blätter von in Töpfen gewachsenen Weizensämlingen der Sorte "Kanzler" wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22°C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropfnässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22°C und 65 bis 70 % relativer Luftfeuchte für 7 Tage kultiviert. Dann wurde das Ausmaß der Rostpilzentwicklung auf den Blättern ermittelt.

In diesem Test zeigten die mit 250 mg/l der Verbindungen 9.7, 9.8, 9.10, 9.12 und 9.13 aus Tabelle 9 behandelten Pflanzen maximal 20 % Befall, während die unbehandelten Pflanzen zu 90 % befallen waren.

## Patentansprüche

1. Pyrazolcarbonsäureanilide der Formel I 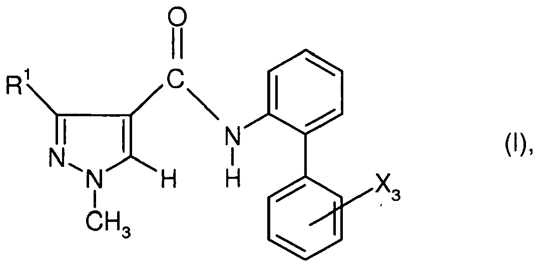 in der
X Fluor oder Chlor, wobei die Reste X verschiedene Bedeutungen haben können,und
R¹ Difluormethyl oder Trifluormethyl
bedeuten.

2. Pyrazolcarbonsäureanilide der Formel I nach Anspruch 1, ausgewählt aus der Gruppe bestehend aus
1-Methyl-3-trifluormethyl-1H-pyrazol-4-carbonsäure(3',4',5'-trifluorbiphenyl-2-yl)-amid,
1-Methyl-3-trifluormethyl-1 H-pyrazol-4-carbonsäure(2',4',5'-trifluorbiphenyl-2-yl)-amid,
1-Methyl-3-trifluormethyl-1H-pyrazol-4-carbonsäure(2',3',4'-trifluorbiphenyl-2-yl)-amid,
1-Methyl-3-difluormethyl-1 H-pyrazol-4-carbonsäure(3',4',5'-trifluorbiphenyl-2-yl)-amid und
1-Methyl-3-difluormethyl-1 H-pyrazol-4-carbonsäure(2',4',5'-trifluorbiphenyl-2-yl)-amid.

3. Mittel zur Bekämpfung von Schadpilzen, enthaltend eine fungizide Menge einer Verbindung der Formel I gemäß Anspruch 1 oder 2 und mindestens einen inerten Zusatzstoff.

4. Mittel nach Anspruch 3 enthaltend zusätzlich einen weiteren Wirkstoff.

5. Verfahren zur Bekämpfung von pflanzenpathogenen Schadpilzen, **dadurch gekennzeichnet, dass** man die Schadpilze, ihren Lebensraum und/oder die vor Pilzbefall zu schützenden Materialien, Pflanzen, den Boden oder Saatgüter mit einer fungizid wirksamen Menge einer Verbindung der Formel I gemäß Anspruch 1 oder 2 behandelt.

6. Verwendung der Verbindungen I gemäß Anspruch 1 oder 2 zur Bekämpfung von pflanzenpathogenen Schadpilzen.

7. Saatgut enthaltend eine Verbindung der Formel I gemäß Anspruch 1 oder 2 in einer Menge von 1 bis 1000g/100kg.

## Claims

1. A pyrazolecarboxanilide of the formula I 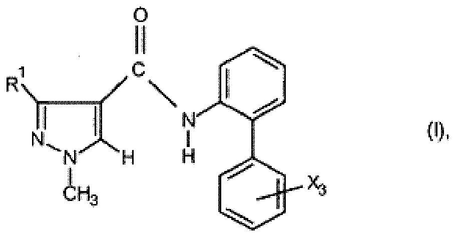 in which
X is fluorine or chlorine, where the radicals X may have different meanings, and
R₁ is difluoromethyl or trifluoromethyl.

2. The pyrazolecarboxanilide of the formula I according to claim 1, selected from the group consisting of
N-(3',4',5'-trifluorobiphenyl-2-yl)-1-methyl-3-trifluoromethyl-1H-pyrazole-4-carboxamide,
N-(2',4',5'-trifluorobiphenyl-2-yl)-1-methyl-3-trifluoromethyl-lH-pyrazole-4-carboxamide
N-(2',3',4'-trifluorobiphenyl-2-yl)-1-methyl-3-trifluoromethyl-1H-pyrazole-4-carboxamide
N-(3',4',5'-trifluorobiphenyl-2-yl)-1-methyl-3-difluoromethyl-1H-pyrazole-4-carboxamide and
N-(2',4',5'-trifluorobiphenyl-2-yl)-1-methyl-3-difluoromethyl-1H-pyrazole-4-carboxamide.

3. A composition for controlling harmful fungi, which composition comprises a fungicidal amount of a compound of the formula I according to claim 1 or 2 and at least one inert additive.

4. The composition according to claim 3, additionally comprising a further active compound.

5. A method for controlling phytopathogenic harmful fungi, which comprises treating the harmful fungi, their habitat and/or the materials, plants, the soil or seed to be protected against fungal attack with a fungicidally effective amount of a compound of the formula I according to claim 1 or 2.

6. The use of the compounds I according to claim 1 or 2 for controlling phytopathogenic harmful fungi.

7. Seed, comprising a compound of the formula I according to claim 1 or 2 in an amount of from 1 to 1000 g/100 kg.

## Revendications

1. Anilides de l'acide pyrazolecarboxylique de formule I 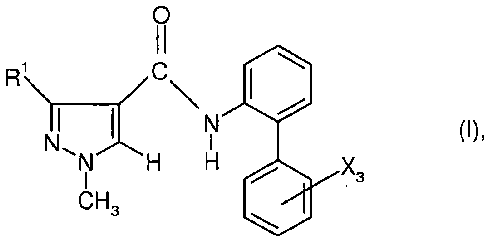 dans laquelle
X est le fluor ou le chlore, les radicaux X pouvant avoir différentes significations, et
R¹ est le groupe difluorométhyle ou trifluorométhyle.

2. Anilides de l'acide pyrazolecarboxylique de formule I selon la revendication 1, choisis dans le groupe consistant en
le (3',4',5'-trifluorobiphényle-2-yl)-amide de l'acide 1-méthyl-3-trifluorométhyl-1H-pyrazole-4-carboxylique,
le (2',4',5'-trifluorobiphényle-2-yl)-amide de l'acide 1-méthyl-3-trifluorométhyl-1H-pyrazole-4-carboxylique,
le (2',3',4'-trifluorobiphényle-2-yl)-amide de l'acide 1-méthyl-3-trifluorométhyl-1H-pyrazole-4-carboxylique,
le (3',4',5'-trifluorobiphényle-2-yl)-amide de l'acide 1-méthyl-3-difluorométhyl-1H-pyrazole-4-carboxylique,
le (2',4',5'-trifluorobiphényle-2-yl)-amide de l'acide 1-méthyl-3-difluorométhyl-1H-pyrazole-4-carboxylique.

3. Produit pour lutter contre les champignons nuisibles, contenant une quantité fongicide d'un composé de formule I selon la revendication 1 ou 2 et au moins un additif inerte.

4. Produit selon la revendication 3, contenant en outre une matière active supplémentaire.

5. Procédé pour lutter contre les champignons nuisibles phytopathogènes, **caractérisé en ce qu'**on traite les champignons nuisibles, leur biotope et/ou les matériaux, les plantes, le sol ou les semences à protéger d'une infestation fongique, avec une quantité à effet fongicide d'un composé de formule I selon la revendication 1 ou 2.

6. Utilisation des composés I selon la revendication 1 ou 2 pour lutter contre les champignons nuisibles phytopathogènes.

7. Semence contenant un composé de formule I selon la revendication 1 ou 2 en une quantité de 1 à 1000 g/100 kg.
